(19) Europäisches Patentamt
European Patent Office
Office européen des brevets

(11) EP 2 374 869 B1

(12) EUROPEAN PATENT SPECIFICATION

(45) Date of publication and mention of the grant of the patent:
**14.08.2013 Bulletin 2013/33**

(21) Application number: **09830560.0**

(22) Date of filing: **01.12.2009**

(51) Int Cl.:
***C12N 1/20*** (2006.01) ***C12N 1/00*** (2006.01)

(86) International application number:
**PCT/KR2009/007098**

(87) International publication number:
**WO 2010/064822 (10.06.2010 Gazette 2010/23)**

(54) **EFFECTIVE CONTROL OF VIRAL PLANT DISEASE WITH STRAINS OF PSEUDOMONAS OLEOVORANS**

WIRKSAME PFLANZENSCHÄDLINGSBEKÄMPFUNG MIT PSEUDOMONAS OLEOVORANS-STÄMMEN

LUTTE EFFICACE CONTRE UNE MALADIE VIRALE DES PLANTES AVEC DES SOUCHES DE PSEUDOMONAS OLEOVORANS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **02.12.2008 KR 20080120899**

(43) Date of publication of application:
**12.10.2011 Bulletin 2011/41**

(73) Proprietor: **Lee, Yong Jin**
**Seoul 137-070 (KR)**

(72) Inventors:
- **HWANG, In Cheon**
**Gyeongju-si**
**Gyeongsangbuk-do 780-130 (KR)**
- **JANG, Cheol**
**Gyeongju-si**
**Gyeongsangbuk-do 780-130 (KR)**
- **KIM, Nam Gyu**
**Daejeon 300-430 (KR)**
- **KIM, Hyeong Min**
**Gyeongju-si**
**Gyeongsangbuk-do 780-050 (KR)**
- **LEE, Yong Jin**
**Seoul 137-070 (KR)**
- **LEE, Byung Man**
**Seoul 137-764 (KR)**

(74) Representative: **Rowe, Daniel**
**Dehns**
**St Bride's House**
**10 Salisbury Square**
**London**
**EC4Y 8JD (GB)**

(56) References cited:
**KR-B1- 100 545 385 KR-B1- 100 817 548**
**US-A- 6 156 560**

- **MAURHOFER M ET AL: "Induction of systemic resistance of tobacco to tobacco necrosis virus by the root-colonizing Pseudomonas fluorescens strain CHA0: Influence of the gacA gene and of pyoverdine production", PHYTOPATHOLOGY, vol. 84, no. 2, 1994, pages 139-146, XP002673963, ISSN: 0031-949X**
- **RAUPACH G S ET AL: "INDUCED SYSTEMIC RESISTANCE IN CUCUMBER AND TOMATO AGAINST CUCUMBERMOSAIC CUCUMOVIRUS USING PLANT GROWTH-PROMOTING RHIZOBACTERIA (PGPR)", PLANT DISEASE, AMERICAN PHYTOPATHOLOGICAL SOCIETY, ST. PAUL, MN, US, vol. 80, no. 8, 1 January 1996 (1996-01-01), pages 891-894, XP000916247, ISSN: 0191-2917**
- **MURPHY JOHN F ET AL: "Plant growth-promoting rhizobacterial mediated protection in tomato against Tomato mottle virus", PLANT DISEASE, vol. 84, no. 7, July 2000 (2000-07), pages 779-784, XP002673964, ISSN: 0191-2917**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **VIRGINIA O. STOCKWELL ET AL.: 'Using Pseudomonas spp. for Integrated Biological Control' PHYTOPATHOLOGY vol. 97, 31 December 2007, pages 244 - 249**
- **JIMENA A. RUIZ ET AL.: 'Polyhydroxyalkanoate Degradation Is Associated with Nucleotide Accumulation and Enhances Stress Resistance and Survival of Pseudomonas oleovorans in Natural Water Microcosms' APPL. ENVIRON. MICROBIOL. vol. 67, no. 1, 31 January 2001, pages 225 - 230**

**Description**

## FIELD OF THE INVENTION

The present invention relates to a *Pseudomonas oleovorans* strain having a controlling activity against plant viral diseases; and a microbial agent for controlling plant viral diseases comprising the same.

## BACKGROUND OF THE INVENTION

Total cultivation area of garden crops has been increasing every year, however, various plant viral diseases have been occurred in most of the garden crops, resulting in serious economic damage.

The plant viruses damaging to the garden crops include Cucumber mosaic virus (CMV), Tobacco mosaic virus (TMV) and Potato virus Y (PVY). In order to control these viruses, agronomical controlling methods such as those using disease-free seeds and breeding novel cultivars resistant to the viruses have been developed, but the effects thereby are insignificant.

Meanwhile, transgenic plants resistant to the viruses have been developed by introducing a gene, such as a coat protein gene, a replication-associated gene, a satellite RNA gene and an antisense gene into the plants, but it will take for more time to achieve an industrial success (Fitchen, J. H. and Beachy, R. N., Annu. Rev. Microbiol., 47:739-763, 1993).

Recently, there have been many studies to develop novel microbial agents using microorganisms for controlling the plant viruses. Especially, the microbial agents are environmental-friendly means which is capable of preserving natural ecosystem and has no mammalian toxicity and, therefore, demand for the microbial agents is increasing.

## SUMMARY OF THE INVENTION

Accordingly, it is an object of the present invention to provide a microorganism effective in controlling plant viral diseases; and a microbial agent comprising the same.

In accordance with the above object, the present invention provides *Pseudomonas oleovorans* KBPF-004 (KCTC 10159BP) having a controlling activity against plant viral diseases; a culture thereof; a dried powder of the culture; and a microbial agent for controlling plant viral diseases comprising the strain, a culture thereof, or a dried powder of the culture as an active ingredient.

## BRIEF DESCRIPTION OF THE DRAWINGS

The above and other objects and features of the present invention will become apparent from the following description of the invention, when taken in conjunction with the accompanying drawings, which respectively show:

Fig. 1: the phylogenetic tree of the KBPF-004 strain;
Fig. 2: the controlling activity of the 1,000-fold dilution of the KBPF-004 25% wettable powder (WP) against tobacco mosaic virus;
Fig. 3: the inhibiting effect of the KBPF-004 25% WP against tobacco mosaic virus confirmed by RT-PCR;
Fig. 4: the inhibiting effect of the KBPF-004 25% WP against the single- or multiple-infection of viruses confirmed by RT-PCR (C: Chungyang red pepper; D: Daemyung pepper; M: size marker; H: healthy plant; N: non-treated group; and 1, 2: KBPF-004-treated groups);
Fig. 5: the photography of the virus particles treated with KBPF-004 25% WP confirmed by electron microscopy (TMV: Tobacco mosaic virus and PVY: Potato virus Y);
Fig. 6: the infection inhibiting effect of KBPF-004 25% WP against the tobacco mosaic virus;
Fig. 7: the controlling effect of the KBPF-004 25% WP against the pepper mottle virus;
Fig. 8: the controlling effect of the KBPF-004 25% WP against the rice stripe virus (A: non-treated group; and B: 500-fold diluted KBPF-004 25% WP-treated group);
Fig. 9: the controlling effect of the KBPF-004 2.5% granules against the tobacco mosaic virus; and
Fig. 10: the controlling effect of the KBPF-004 70% aqueous suspensions (AS) against the Tomato yellow leaf curl virus (A: non-treated (the commercial insecticide-treated group); and B: the 500-fold dilution of KBPF-004 70% aqueous suspensions and the commercial insecticide-treated group).

## DETAILED DESCRIPTION OF THE INVENTION

The present invention provides *Pseudomonas oleovorans* strain KBPF-004 (KCTC 10159BP) having a controlling activity against plant viral diseases.

*Pseudomonas oleovorans* KBPF-004 (KCTC 10159BP) forms yellow round colonies having pectinated stripes in the outline. The strain is shaped like a rod of 2~3 μm in length and 0.3~0.5 μm in width, and has a single long flagellum.

The inventive strain has 16S rDNA of 1,479 bp nucleotide sequence represented by SEQ ID NO: 1, which has 99.05% sequence homology to that of *Pseudomonas oleovorans* IAM 1508T. The taxonomic position of the inventive strain was investigated by constructing a phylogenetic tree (Fig. 1) according to Clustal method (Thompson JD et al., Nuc. Acid. Res. 25: 4876-82, 1997).

The inventive strain is different from the existing *Pseudomonas oleovorans* strains in terms of its specific anti-viral activities.

Therefore, the strain was designated as *Pseudomonas oleovorans* KBPF-004, and deposited on January 11, 2002 at Korean Collection for Type Cultures (KCTC) under the accession number of KCTC 10159BP.

The present invention also provides a culture of *Pseudomonas oleovorans* KBPF-004 (KCTC 10159BP) effective in controlling plant viral diseases.

The culture may be prepared by inoculating *Pseudomonas oleovorans* KBPF-004 (KCTC 10159BP) cells onto a medium and subjecting them to a fermentation.

The medium may comprise conventional ingredients of a medium for culturing gram negative bacteria without any limitation. Preferably, the medium may comprise 1 g to 20 g of glucose, 1 g to 30 g of yeast extract, 0.1 g to 1 g of magnesium sulfate, 0.5 g to 5 g of potassium dihydrogen phosphate and 0.5 g to 5 g of potassium monohydrogen phosphate based on 1 ℓ of water. More preferably, the medium may comprise 7 g to 14 g of glucose, 15 g to 25 g of yeast extract, 0.1 g to 0.2 g of magnesium sulfate, 1 g to 2 g of potassium dihydrogen phosphate and 1 g to 2 g of potassium monohydrogen phosphate based on 1 ℓ of water.

The fermentation may be conducted at a temperature of 20°C to 40°C, preferably, 26 °C to 34 °C, with an aeration rate of 50 ℓ/min to 200 ℓ/min, preferably, 100 ℓ/min to 120 ℓ/min, and a rotation speed of 100 rpm to 250 rpm, preferably 120 rpm to 200 rpm. In order to prepare the inventive microbial agent, it is preferred to employ both strain cells and culture filtrates.

Further, the present invention provides a dried powder of the culture.

The dried powder may be prepared by the steps of sterilization, concentration and pulverization (freeze drying and milling).

The sterilization step may be conducted by heating the cell culture, upon completion of culturing, at a temperature of 80 °C to 120°C for 1 to 20 min, preferably, at 90 °C to 100°C for 5 to 10 min.

The concentration step may be conducted by concentrating the sterilized culture under a reduced pressure at a temperature of 40 °C to 80°C for 12 to 48 hours, preferably, at 65 °C to 75 °C for 24 to 36 hours.

The pulverization step may be conducted, but are not limited to, by subjecting the concentrated culture to drying process by freeze drying or spray-drying, and them to milling to prepare a powdered culture.

The freeze drying process may be conducted by drying the concentrated culture with sequentially rising a temperature starting from -80°C for 48 to 96 hours, preferably, 60 to 80 hours.

The milling process is conducted by grinding the resulting freeze-dried products using a pin mill grinder to have particle sizes of 2 mm or below.

A microbial agent may be prepared by formulating the cells, the culture or the dried powder of the culture in combination with surfactants, nutrients, and carriers.

In addition, the present invention provides a microbial agent for controlling plant viral diseases comprising *Pseudomonas oleovorans* KBPF-004 (KCTC 10159BP) cells, a culture thereof, or a dried powder of the culture as an active ingredient.

The microbial agent for controlling plant viral diseases exhibits an anti-viral activity depending on the concentration of the active ingredient.

The microbial agent of the present invention may comprise 2.5 to 70% by weight of *Pseudomonas oleovorans* KBPF-004 (KCTC 10159BP) cells, the culture thereof, or the dried powder of the culture; 2 to 30% by weight of a surfactant; and a residual amount of a carrier. Preferably, the microbial agent of the present invention may comprise 2.5 to 70% by weight of *Pseudomonas oleovorans* KBPF-004 (KCTC 10159BP) cells, the culture thereof, or the dried powder of the culture.

The surfactants may include an anionic surfactant, a nonionic surfactant, or a mixture thereof. The surfactants may be selected from the group consisting of sodium or calcium salts of sulfonate compounds such as $C_{8\sim12}$ alkylaryl sulfonate, $C_{8\sim12}$ dialkylaryl sulfonate, $C_{8\sim12}$ dialkyl sulfosuccinate, lignin sulfonate, naphthalene sulfonate condensates, naphthalene sulfonate formalin condensates, $C_{8\sim12}$ alkyl naphthalene sulfonate formalin condensates and polyoxyethylene $C_{8\sim12}$ alkylphenyl sulfonate; sodium or calcium salts of sulfate compounds such as $C_{8\sim12}$alkyl sulfate, $C_{8\sim12}$ alkylaryl sulfate, polyoxyethylene $C_{8\sim12}$ alkyl sulfate and polyoxyethylene $C_{8\sim12}$ alkylphenyl sulfate; sodium or calcium salts of succinate compounds such as polyoxyalkylene succinate; anionic surfactants such as sodium benzoate and alkyl carboxylate; nonionic surfactants such as polyoxyethylene $C_{12\sim18}$ alkyl ether, polyoxyethylene $C_{8\sim12}$ alkylphenyl

ether, polyoxyethylene $C_{8\sim12}$ alkylphenyl polymers and ethyleneoxide propyleneoxide copolymers; polycarboxylate; Triton® 100; Tween® 80; and a mixture thereof, but are not limited thereto.

The carriers may be selected from the group consisting of bentonite, talc, clay, kaolin, calcium carbonate, silica, pumice stone, diatomaceous earth, acidic white bole, zeolite, perlite, white carbon, ammonium sulfate, urea, glucose, dextrin, water, and a mixture thereof, but are not limited thereto.

Also, the microbial agent of the present invention may be formulated by using the surfactants and/or carriers in a form selected from the group consisting of wettable powders (WP), water dispersible granules (WG), suspension concentrates (SC), granules, aqueous suspensions (AS), soluble powders (SP), water soluble granules (SG), capsules.

In the present invention, the cells or the culture may be supplied in the form of the microbial agent for controlling plant viral diseases comprising the same, or in a separate storage form for a long-term storage and for mixing with other ingredients before use. For long-term storage, the cells or cultures may be stored in a glycerol storage solution at below -70 °C , or in a freeze-dried form.

The wettable powder, a form of the microbial agent provided in the present invention, may be prepared by obtaining a dried powder of the culture by a post-treatment process; adding the surfactants, nutrients and carriers thereto; and mixing them.

The granules, a form of the microbial agent provided in the present invention, may be prepared by obtaining a dried powder of the culture by a post-treatment process; adding the surfactants, carriers and disintegrating agents thereto; and mixing them. The disintegrating agent useful in the present invention may be selected from the group consisting of bentonite, talc, dialite, kaolin, calcium carbonate and a mixture thereof.

The granules may further comprise an ingredient selected from the group consisting of surface active agents, inactive carriers, preservatives, wetting agents, supply-promoting agents, attracting agents, encapsulants, binders, emulsifiers, dyes, UV protectors, buffering agents or flow agents, in addition to the microbial cells and/or the fermentation products (the culture of the microorganism).

The aqueous suspensions, a form of the microbial agent provided in the present invention, may be prepared by sterile-concentrating the culture by a post-treatment process; adding an ingredient selected from the group consisting of the surfactants, preservatives, wetting agents, supply-promoting agents, attracting agents, UV protectors and buffering agents thereto; and mixing them.

The microbial agent of the present invention is effective in controlling most of the plant viral diseases caused by pathogenic virus groups, such as Allexivirus, Alfamovirus, Ampelovirus, Bymovirus, Begomovirus, Capillovirus, Carlavirus, Carmovirus, Caulimovirus, Closterovirus, Comovirus, Cucumovirus, Crinivirus, Cytorhabdovirus, Fabavirus, Flexiviridae, Foveavirus, Furovirus, Geminivirus, Hordeivirus, Ilarvirus, Luteovirus, Maculavirus, Nepovirus, Potexvirus, Potyvirus, Phytoreovirus, Polerovirus, Pomovirus, Sadwavirus, Taastrupvirus, Tenuivirus, Tobamovirus, Tobravirus, Tombusvirus, Tospovirus, Trichovirus, and a combination thereof. Especially, the inventive microbial agent is very effective in controlling Tobamovirus, Potyvirus and Tenuivirus groups composed of bar-shaped (thread-shaped) single stranded RNA, Cucumovirus group composed of circular single stranded RNA and Begomovirus group composed of circular single stranded DNA.

More specifically, the microbial agent of the present invention presents an excellent controlling effect on Pepper mottle virus (PepMoV), Pepper mild mottle virus (PMMoV), Cucumber mosaic virus (CMV), Tomato yellow leaf curl virus (TYLCV), Cucumber green mottle mosaic virus (CGMMV), Potato virus Y (PVY), Zucchini yellow mosaic virus (ZYMV), Turnip mosaic virus (TuMV), or Rice stripe virus (RSV) and the like.

The following Examples are intended to further illustrate the present invention without limiting its scope.

**Example 1: Isolation and identification of KBPF-004**

<u><1-1> Isolation of KBPF-004 strain</u>

Soil samples comprising tobacco plant roots were collected from the tobacco fields located in eumsung-gun of chungcheongbuk-do. The collected soil sample was diluted with sterile distilled water, and the dilution was spread onto a TSA agar medium (Difco, Detroit, MI) supplemented with 100 ppm cyclohexamide and incubated at 27 °C to isolate a pure culture of a strain.

<u><1-2> Identification of KBPF-004 strain</u>

The strain isolated in Example <1-1> was incubated in a Mueller Hinton medium comprising 2.0 g of beef extract, 17.5 g of casein, 1.5 g of starch and 17.5 g of agar at 30 °C for 24 hours.

Yellow round colonies of the isolated strain were formed, which had pectinated stripes in the outline. The strain was shaped like a rod of 2~3 μm in length and 0.3~0.5 μm in width, and had a single long flagellum. In order to identify biochemical features of the strain, fatty acid composition analysis, Biolog GN microplate analysis and API20NE analysis were conducted, and the results are shown in Tables 1 to 3, respectively.

<Table 1>

| Fatty acid composition | Content (%) |
|---|---|
| $C_{10:0}$ 3-OH | 2.19 |
| $C_{12:0}$ | 5.14 |
| $C_{12:0}$ 2-OH | 2.70 |
| $C_{12:0}$ 3-OH | 3.79 |
| $C_{16:1}$ w7c/$_{15:0}$ *iso* 2-OH | 18.74 |
| $C_{16:0}$ | 20.45 |
| $C_{18:1}$ w7c | 45.88 |

<Table 2>

| | | | | | |
|---|---|---|---|---|---|
| - Water | - α-cyclodextrin | - Detrin | - glycogen | - Tween 40 | - Tween 80 |
| - i-erythritol | + D-fructose | + L-fucose | + D-galactose | -gentiobiose | + D-glucose |
| - D-melibiose | - β-methyl-D glucoside | - D-psicose | - D-raffinose | - L-rhamnose | + D-sorbitol |
| - Acetic acid | + $C_{is}$-aconitic acid | + Citric acid | - Fomic acid | + D-galactonic acid lactone | + D-galacturonic acid |
| - p-hydroxy phenylacetic acid | v Itaconic aicd | - α-keto butyric acid | + α-keto glutaric acid | - α-keto valeric acid | + D,L-lactic acid |
| + Bromo succinic acid | - Succinamic acid | - Glucuron amide | - Alaninamide | v D-alanine | + L-alanine |
| - L-histidine | - Hydroxy L-proline | - L-leucine | - L-ornithine | - L-pheyl alanine | + L-proline |
| v Urocanic acid | - Inosine | -Uridine | - Thymidine | - Phenyl ethylamine | - Putrescine |
| - N-acetyl-D-galactosamine | - N-acetyl-D-glucosamine | - Adonitol | + L-arabinose | + D-arabitol | - cellobiose |
| + m-inositol | - D-lactose | - Lactulose | - Maltose | + Mannitol | + D-mannose |
| - Sucrose | + D-trehalose | - Turanose- | - Xylitol- | + Methyl pyruvate | + Mono-methyl succinate |
| + D-gluconic acid | + D-glucosaminic acid | + D-glucuronic acid | - α-hydroxybutyric acid | + β-hydroxybutyric acid | -γ-hydroxybutyric acid |
| v Malonic acid | - Propioic acid | + Quinic acid | + D-saccharicenyl acid | - Sebacicc acid | + Succinic acid |
| - L-alanyl-glycine | + L-asparagine | + L-aspartic acid | + L-glutamic acid | - Glycyl-L-asparatic acid | - Glycy-L-glutamic acid |
| + L-pyroglutamic acid | -D-serine- | + L-serine | -L-threonine - | v D,L-camitine | - γ-aminogutyric acid |
| - 2-amino ethanol | - 2,3-butanediol | + Glycerol | - D,L-glycerol phosphate | - Glucose-1-phosphate | - Glucose-6-phosphate |
| Degree of reaction | | | | | |

(continued)

| +: at least 80%; -: at most 20%; v: 21-79% |
|---|

<Table 3>

| Test | Reaction / Enzyme | Result |
|---|---|---|
| $NO_3$ | Reduction of nitrates to nitrites | - |
| | Reduction of nitrates to nitrogen | - |
| TRP | Indole production | - |
| GLU | Acidification | - |
| ADH | Arginine dihydrolase | - |
| URE | Urease | - |
| ESC | Hydrolysis (β-glucosidase) | - |
| GEL | Hydrolysis(protease) | - |
| PNG | β-glucosidase | - |
| GLU | Glucose assimilation | + |
| RAR | Arabinose assimilation | + |
| MNE | Mannose assimilation | + |
| MAN | Mannitol assimilation | + |
| NAG | N-acetyl-glucosamine assimilation | - |
| MAL | Maltose assimilation | - |
| GNT | Gluconate assimilation | + |
| CAP | Caprate assimilation | + |
| ADI | Adipate assimilation | - |
| MLT | Malate assimilation | + |
| CIT | Citrate assimilation | + |
| PAC | Phenyl-acetate assimilation | - |
| OX | Cytochrome oxidase | + |

Also, the inventive strain was identified based on 16S rDNA nucleotide sequencing. The isolated strain has 16s rDNA of 1,479 bp nucleotide sequence (SEQ ID NO: 1) which shows 99.05% sequence homology to that of *Pseudomonas oleovorans* IAM 1508T strain. The taxonomic position of the inventive strain was determined by constructing a phylogenetic tree (Fig. 1) according to Clustal method (Thompson JD et al., Nuc. Acid. Res. 25: 4876-82, 1997).

The strain was designated as *Pseudomonas oleovorans* KBPF-004, and deposited on January 11, 2002 at Korean Collection for Type Cultures (KCTC) under the accession number of KCTC 10159BP.

<1-3> Specificity of KBPF-004 strain having anti-viral activities

KBPF-004 strain and *Pseudomonas oleovorans* ATCC 8062 as a type strain thereof were cultured in the Mueller Hinton medium with shaking at 30 °C for 24 hours. The obtained culture was diluted 20-fold and the anti-viral activities thereof were examined according to a tobacco half-leaf method (Kim et al., Plant Pathol. J. 20(4): 293-296, 2004).

The tobacco half-leaf method is a method employing a phenomenon that *Nicotiana tabacum* cv. Xanthi nc as a local lesion host plant forms black spots due to the cell necrosis around the Tobacco mosaic virus-infected regions by the resistance gene of the Tobacco mosaic virus. Specifically, a test material for examining anti-viral activity was sprayed onto half-leaves of the upper $3^{rd}$ and $4^{th}$ leaves of the local lesion host plant at 7 foliage leaf stages. Then, carborundum (abrasive) was evenly applied to the $3^{rd}$ and $4^{th}$ leaves and a dilution of the tobacco mosaic virus was applied thereto for the examination of the anti-viral activity of the test material. The applied carborundum induces scratches on the tobacco leaves and the tobacco mosaic virus pass into the host plants through the scratches. After 3-4 days from the inoculation, black spots were found in the infected leaves.

Control values of the test materials were calculated by the following formula and the results are shown in Table 4.

$$\text{Control value (\%)} = [1-(\text{Number of black spots in the strain-treated half-leaves} / \text{Number of black spots in the non-treated half-leaves})] \times 100$$

<Table 4>

| | Strain | Anti-viral activity |
|---|---|---|
| Type strain | *Pseudomonas oleovorans* ATCC 8062 | 19.7% |
| KBPF-004 strain | *Pseudomonas oleovorans* KCTC 10159BP | 96.0% |

As shown in Table 4, KBPF-004 strain has a specifically high anti-viral activity, which demonstrates that KBPF-004 strain is a specific strain having a different anti-viral activity compared with the existing *Pseudomonas oleovorans* strains.

<1-4> Specificity of anti-viral active materials of KBPF-004

It is known that *Pseudomonas oleovorans* species produce polyhydroxy alkanoate (PHA) as a biodegradable polymer. In order to identify whether the specific anti-viral activity of KBPF-004 strain is induced by PHA or not, the anti-viral activity of PHA (sigma) and a culture of KBPF-004 strain was respectively determined according to the tobacco half-leaf method, and the results are shown in Table 5.

<Table 5>

| | Anti-viral activity |
|---|---|
| PHA | 0.0% |
| KBPF-004 strain culture | 97.0% |

As shown in Table 5, PHA exhibits no anti-viral activity, whereas KBPF-004 strain culture exhibits a high anti-viral activity. These results prove that the anti-viral active materials produced by KBPF-004 strain are different from PHA.

Further, in order to identify whether the anti-viral active materials produced by KBPF-004 strain also exhibits an antibiotic effect or not, the paper disk analysis was conducted using seven plant pathogenic fungi and four antibiotic supersensitive strains to check the formulation of inhibition circles. The results are shown in Table 6.

<Table 6>

| | Scientific name | Activity |
|---|---|---|
| Plant pathogenic fungi | *Alternaria mali* | - |
| | *Botrytis cinerea* | - |
| | *Colletotrichum gloeosporioides* | - |
| | *Magnaporthe grisea* | - |
| | *Fusarium oxysporum* | - |
| | *Phytophthora capsici* | - |
| | *Rhizoctonia solani* | - |

(continued)

| | Scientific name | Activity |
|---|---|---|
| Antibiotic supersensitive strain | *E.coli* KCTC 1682 | - |
| | *E.coli* KCTC 1683 | - |
| | *E.coli* KCTC 1923 | - |
| | *E.coli* KCTC 1924 | - |

As shown in Table 6, both the plant pathogenic fungi and antibiotic supersensitive strains grow without inhibition by the KBPF-004 strain culture. It demonstrates that the anti-viral active materials produced by KBPF-004 strain are different from the existing antibiotic materials.

**Example 2: Preparation of optimized medium for producing anti-viral active materials**

In order to determine a medium condition for producing anti-viral active materials superior to the Mueller Hinton medium, a variety of carbon and nitrogen sources were employed for preparing an optimized medium.

Specifically, the strain was subjected to shaking culture at 30 °C for 24 hours by using dextrin, glucose, glycerol, sucrose or water-soluble starch as a carbon source, and peptone, yeast extract, casein, wheat bran extract, ammonium dihydrogen phosphate or ammonium sulfate as a nitrogen source. The optical density (O.D) of the obtained culture was measured at 600 nm, and the anti-viral activity was determined by the tobacco half-leaf method. The results are shown in Table 7.

<Table 7>

| Medium | Anti-viral activity (1/40 dilution) | Optical density (660 nm) |
|---|---|---|
| Mueller Hinton medium | 57% | 9.2 |
| Optimized medium | 92% | 23.2 |

As shown in Table 7, the optimized medium supplemented with 1 to 5% by weight of glucose as a carbon source and 1 to 5% by weight of yeast extract as a nitrogen source presents a significantly improved strain growth and anti-viral activity compared with the Mueller Hinton medium.

**Example 3: Preparation of a culture and a dried powder of KBPF-004 strain**

<u><3-1> Preparation of a culture of KBPF-004 strain</u>

In order to prepare a culture of KBPF-004 strain on a large scale, a medium supplemented with 10 g of glucose, 20 g of yeast extract, 0.2 g of magnesium sulfate, 2 g of potassium dihydrogen phosphate and 2 g of potassium hydrogen phosphate based on 1 ℓ of water was employed.

A fermentation process for production on a large scale was conducted by employing a pilot-scale fermentor. 30 ℓ of strain culture was fermented in a 50 ℓ fermentor and the resulting culture was transported to a 500 ℓ fermentor upon terminating the culturing. Along the same lines, 3,000 ℓ of strain culture was fermented in a 5,000 ℓ fermentor. The fermentation process was conducted at a temperature of 30°C, with an aeration rate of 1VVM (volume of air added to liquid volume per minute), and a rotation speed of 200 rpm (50 ℓ fermentor), 150 rpm (500 ℓ fermentor) or 80 rpm (5,000 ℓ fermentor) for 1 day to prepare the KBPF-004 strain culture.

<u><3-2> Preparation of a dried powder of KBPF-004 strain culture</u>

In order to prepare a dried powder of the KBPF-004 strain culture, post-treatment process comprising strain sterilization, concentration, freeze-drying and milling was conducted.

The strain sterilization process was conducted by heating the fermentor at 80 °C for 20 min after the culturing to kill the cells. The concentration process was conducted by concentrating the resulting culture under a reduced pressure at 65°C for 32 hours. The freeze-drying process was conducted by heating the concentrated culture with gradually raising the temperature starting from -80 °C for 52 hours. The milling process was conducted by grinding the freeze-dried products using a pin mill grinder to prepare a dried powder having a particle size of below 2 mm.

**Example 4: Preparation of microbial agents using products of KBPF-004 strain culture**

Microbial agents of KBPF-004 strain such as wettable powders (WP), water dispersible granules (WG), suspension concentrates (SC) and granules were prepared by using the dried powder of the KBPF-004 strain culture, and aqueous suspensions (AS) were prepared by using the aqueous KBPF-004 strain culture.

<4-1> Preparation of a wettable powder using dried powder of the KBPF-004 strain culture

In order to formulate a wettable powder from the dried powder of the KBPF-004 strain culture, 25% by weight of the dried powder of the KBPF-004 strain culture obtained in <3-2> was mixed with 3% by weight of polyoxyethylene octylphenylsulfate, 3% by weight of sodium lignin sulfonate, 2% by weight of sodium laurylsulfate, 5% by weight of white carbon and a residual amount of kaolin, and the mixture was ground using a drying type grinding machine. The average particle size of the ground mixture was 6.47 μm. The wettable powder thus obtained was subjected to physicochemical and biological analyses, and the results are shown in <4-6> below.

<4-2> Preparation of water dispersible granule using dried powder of the KBPF-004 strain culture

In order to formulate water dispersible granules from the dried powder the KBPF-004 strain culture, 25% by weight of the dried powder of the KBPF-004 strain culture obtained in <3-2> was mixed with 6% by weight of sodium naphthalene sulfonate formalin condensate, 2% by weight of sodium laurylsulfate, 2% by weight of sodium lignin sulfonate, 5% by weight of diatomaceous earth and a residual amount of calcium carbonate, and the mixture was ground using a drying type grinding machine. The average particle size of the ground mixture was 6.08 μm. Then, 10% by weight of water based on the mixture was added to the mixture, and the resulting mixture was subjected to kneading and molding process using a granulating machine and dried for 30 min in a fluidized bed dryer to obtain the water dispersible granules. The water dispersible granules thus obtained were subjected to physicochemical and biological analyses, and the results are shown in <4-6> below.

<4-3> Preparation of a suspension concentrate using dried powder of the KBPF-004 strain culture

In order to formulate a suspension concentrate from the dried powder the KBPF-004 strain culture, 25% by weight of the dried powder of the KBPF-004 strain culture obtained in <3-2> was mixed with 3% by weight of polyoxyethylene octylphenyl ether, 2% by weight of ethyleneoxide propyleneoxide copolymer, 2% by weight of sodium dioctylsulfosuccinate, 10% by weight of propylene glycol and 55.5% by weight of distilled water, and the resulting mixture was ground using a wet grinding machine. The average particle size of the ground mixture was 2.45 μm. Then, 2.5 % by weight of 2% aqueous xanthan gum prepared by stirring and mixing steps was added to the mixture, and the resulting mixture was stirred and mixed by a mixer to obtain a suspension concentrate in the form of an aqueous suspension. The suspension concentrate thus obtained was subjected to physicochemical and biological analyses, and the results are shown in <4-6> below.

<4-4> Preparation of granules using dried powder of the KBPF-004 strain culture

In order to examine the controlling effect of the KBPF-004 strain when applied to the soils, 2.5% by weight of the dried powder of the KBPF-004 strain culture obtained in <3-2> was mixed with 0.5% by weight of calcium lignin sulfonate and 1.5% by weight of kaolin, and the mixture was ground using a drying type grinding machine to prepare a ground mixture having an average particle size of 6.25 μm. 1% by weight of polyvinyl alcohol and 0.5% by weight of dextrin were dissolved in 1.5% by weight of water to prepare a binding solution. The binding solution was applied to 94% by weight of sand, and the above-prepared mixture was coated thereon. The resulting granules were dried in a fluidized bed dryer for 30 min. The granules thus obtained were subjected to physicochemical and biological analyses, and the results are shown in <4-6> below.

<4-5> Preparation of aqueous suspensions using the KBPF-004 strain culture

In order to formulate aqueous suspensions from the aqueous KBPF-004 strain culture, the aqueous KBPF-004 strain culture obtained in <3-1> was subjected to the post-treatment process comprising the strain killing and concentration, and 70% by weight of the strain culture thus obtained was mixed with 5% by weight of polyoxyethylene octylphenyl ether, 5% by weight of sodium dioctylsulfosuccinate, 10% by weight of propylene glycol and 10 % by weight of ethanol. The resulting mixture was stirred and mixed to obtain the aqueous suspensions. The aqueous suspensions thus obtained were subjected to physicochemical and biological analyses, and the results are shown in <4-6> below.

<4-6> Test of the formulation properties and anti-viral effect against tobacco mosaic virus

The properties (formulation form, appearance, wettability, fineness and storage stability) and the control values according to the tobacco half-leaf method of the formulations obtained in <4-1> to <4-5> were determined and the results are shown in Table 8 (Test method of agricultural chemicals notified by the Korean *Rural development administration*).

<Table 8>

| Example | Properties | | | | | Control value (%) |
|---|---|---|---|---|---|---|
| | Formulation | Appearance | Wettability | Fineness | Storage stability | |
| <4-1> | 25% WP | powder | 58 sec | 99.3% | Good | 95.7 |
| <4-2> | 25% WG | granule | 1 min 10 sec | 99.6% | Good | 85.8 |
| <4-3> | 25% SC | liquid | 20 sec | 99.7% | Good | 90.4 |
| <4-4> | 2.5% granule | granule | - | - | Good | 92.3 |
| <4-5> | 70% AS | liquid | - | - | Good | 91.3 |
| Standard of review | | | About 2 min | 44 $\mu$m, at least 98% | 50°C, 4 weeks | Tobacco half-leaf method |

As a result, the five formulations showed suitable physical properties and anti-viral effect according to the standard of review of agricultural chemicals by enforcement regulations of the Korean agrochemical management law. Among formulations, 25% wettable powder obtained in <4-1> showed the most excellent anti-viral effect, therefore, the KBPF-004 25% wettable powder, 2.5% granule and 70% AS were employed in the following tests.

<4-7> Test for aging stability of 25% wettable powder

In order to test the aging stability of the 25% wettable powder obtained in <4-1>, the degree of loss of the anti-viral activity of the wettable powder was determined by the tobacco half-leaf method every one week while storing at 54°C for at least 6 weeks, and the results are shown in Fig. 2. As shown in Fig. 2, the inventive wettable powder exhibited the anti-viral activity on the tobacco mosaic virus at 54 °C for at least 6 weeks. Therefore, a three year warranty of efficacy was set for the inventive wettable powder according to the registration test guidelines and methods of agricultural chemicals notified by the Korean Rural development administration.

**Example 5: Test for inhibiting effect of KBPF-004 25% wettable powder on plant viruses**

<5-1> Concentration-dependent inhibiting effect on tobacco mosaic virus infection

In order to examine the inhibiting effect of the KBPF-004 25% wettable powder on Tobacco mosaic virus-infection according to its concentrations, RT-PCR (Reverse transcriptase-polymerase chain reaction) was performed on the tobacco leaves using primers recognizing a specific nucleotide sequence of virus genomic RNA (protein coat region) (Choi et al., Plant Pathol. J., 14: 7-12, 1998; Yoon, Doctoral Thesis, Seoul Women's Univ., Seoul, 166, 2003) and the results are shown in Fig. 3.

As shown in Fig. 3, no Tobacco mosaic virus was detected when the tobacco leaves were treated with KBPF-004 25% wettable powder in an amount ranging from 0.005 g/ml to 0.02 g/ml. This result shows that KBPF-004 strain effectively inhibits the Tobacco mosaic virus infection.

<5-2> Controlling effect on plant viral infection

In order to examine the controlling effect of the KBPF-004 25% wettable powder on single and complex infection of the plant viruses, 1,000-fold dilution of the KBPF-004 25% wettable powder obtained in <4-1> was sprayed on Chungyang red peppers using a sprayer. Then, Pepper mottle virus (PepMoV), Pepper mild mottle virus (PMMoV) and Cucumber mosaic virus (CMV) were inoculated thereto respectively or in combination, and RT-PCR was conducted to identify whether the RT-PCR products were detected or not. The results are shown in Fig. 4.

As shown in Fig. 4, the Chungyang red peppers inoculated with PepMoV only showed no virus up to 30-40 days regardless of their species, and the Chungyang red peppers inoculated with Pepper mottle virus + Cucumber mosaic virus showed no virus up to 30 days.

Also, the Pepper mottle virus, Pepper mottle virus + Pepper mild mottle virus, and Pepper mottle virus + Cucumber mosaic virus samples to which the anti-viral activities were confirmed in RT-PCR analysis were analyzed using ELISA (enzyme-linked immunosorbent assay)(Clark, et al., J. Gen. Virol. 34: 475-483, 1977) and the results are shown in Table 9.

<Table 9>

| Infected virus | Antiserum | The days after inoculation | Absorbance ($A_{405nm}$) | | |
|---|---|---|---|---|---|
| | | | KBPF-004 treated group | Non-treated group | Healthy plant |
| PepMoV (single infected) | PepMoV | 10 | 0.202 | 0.981 | 0.232 |
| | | 30 | 0.210 | 1.900 | - |
| PepMoV + PMMoV (complex infected) | PepMoV | 10 | 0.173 | 1.200 | 0.243 |
| | | 30 | 0.788 | 1.876 | - |
| | PMMoV | 10 | 0.836 | - | 0.243 |
| | | 30 | 1.504 | 1.366 | - |
| PepMoV + CMV (complex infected) | PepMoV | 10 | 0.310 | - | 0.187 |
| | | 30 | 0.286 | - | - |
| | CMV | 10 | 0.457 | 1.964 | 0.187 |
| | | 30 | 0.382 | - | - |

As shown in Table 9, the controlling effects of the inventive KBPF-004 strain were significantly shown in the molecular and antiserum levels.

<5-3> Infection inhibiting effect on virus genome RNA

In order to examine the inhibiting effect of the KBPF-004 25% wettable powder on the infection by Pepper mild mottle virus (PMMoV) genomic RNA, 1,000-fold dilution of the KBPF-004 25% wettable powder obtained in <4-1> was sprayed on tobacco (*Nicotiana glutinosa*) plants. Then, the genome RNA of the pepper mild mottle virus was inoculated to the plants, and the infection inhibiting effects were determined and the results are shown in Table 10.

<Table 10>

| Treatment | | lesions / 3 half-leaves * | Inhibition (%) |
|---|---|---|---|
| Non-treated group | PMMoV-RNA+phosphate buffer | 531 | 0 |
| KBPF-004 | PMMoV-RNA+phosphate buffer | 3 | 99.4 |
| *the number of lesions formed in 3 half-leaves of tobacco (*Nicotiana glutinosa*) | | | |

As shown in Table 10, the KBPF-004 25% wettable powder-treated group shows no virus infection, whereas the non-treated group shows PMMoV infection, which suggests the KBPF-004 strain directly influences on the virus genome RNA.

<5-4> Resistance inducing effect

In order to examine the resistance inducing effect of the KBPF-004 25% wettable powder, 1,000-fold dilution of the KBPF-004 25% wettable powder was sprayed on the lower leaves of two tobacco species (*Nicotiana tabacum* cv. Xanthi nc and *Nicotiana glutinosa*). After 10 days, the Pepper mild mottle virus was inoculated thereto. The control values were calculated and the results are shown in Table 11.

<Table 11>

| Treatment | Host plant | Number of lesions (Control value) | | |
|---|---|---|---|---|
| | | Treated leave (lower leave) | 1st upper leave | 2nd upper leave |
| KBPF-004 | *Nicotiana tabacum* cv. Xanthi nc | 51 (71.0%) | 96 (55.9%) | 78 (57.3%) |
| | *Nicotiana glutinosa* | 83 (74.8%) | 118 (45.8%) | 169 (46.6%) |
| Non-treated group | *Nicotiana tabacum* cv. Xanthi nc | 176 | 218 | 183 |
| | *Nicotiana glutinosa* | 330 | 349 | 317 |

As shown in Table 11, in the KBPF-004 25% wettable powder-treated group, the controlling effect on Pepper mild mottle virus was shown in the 1st and 2nd upper leaves as well as the treated leaves (lower leaves). Therefore, it is shown that the KBPF-004 25% wettable powder has a resistance inducing effect as well as the direct inhibiting effects on the virus infection and reproduction.

<u><5-5> Virus inhibiting, effect confirmed by an electron microscopy</u>

In order to confirm the direct virus inhibiting effect of the inventive microbial agent, effect of KBPF-004 strain on virus particles or viral coat protein was analyzed by an electron microscopy.

Tobacco mosaic virus (TMV) and Potato virus Y (PVY) were respectively inoculated on a host plant for proliferation (*Nicotiana benthamiana*). After 2 weeks, 50 g of the systemically infected leaves was collected and purified for the virus particles, respectively. Then, the purified virus particles were mixed with 1/100 dilution of the KBPF-004 25% wettable powder (1,000 ppm) in the ratio of 1:1 (v/v), and the resulting mixture was analyzed by an electron microscopy. The results are shown in Fig. 5.

As shown in Fig. 5, it is found that the length of the virus particles was short in the KBPF-004 25% wettable powder-treated group, which suggests that the particles of the TMV or PVY are segmented by the KBPF-004 strain.

Further, the segmented virus particle samples obtained in the above were subjected to the tobacco half-leaf method to examine whether the viral activity is maintained or not, and the results are shown in Fig. 6. As a result, the number of segmented virus particles was increased depending on the strain-treated time, and the number of infected lesions of the host plant was rapidly decreased.

**Example 6: Efficacy of KBPF-004 strain on the major plant viral diseases**

<u><6-1> Efficacy screening</u>

In order to evaluate the efficacy of the microbial agent of KBPF-004 strain on the major plant viruses, an efficacy screening was conducted on a variety of plants in greenhouse pots.

The screening was conducted on seedlings (young plant), and adult plants, if necessary, according to a common method for screening plant viral diseases.

For the efficacy screening, Tobamovirus group such as Tobacco mosaic virus, Cucumber green mottle mosaic virus and Pepper mild mottle virus; Potyvirus group such as Pepper mottle virus, Zucchini yellow mosaic virus, Potato virus Y and Watermelon mosaic virus; Cucumovirus group such as Cucumber mosaic virus were employed as plant viruses, and tobacco, pepper, zucchini, cucumber, watermelon, pumpkin, cabbage and melon were employed as host plants.

Specifically, 250-, 500- and 1,000-fold dilutions of the KBPF-004 25% wettable powder obtained in <4-1> were sprayed on the host plants in an amount of 20L/1,000 $m^2$, respectively, and 1,000-fold dilutions of the respective plant virus were inoculated thereto. The efficacy of the microbial agent was evaluated by the result of the tobacco half-leaf method for Tobacco mosaic virus, and by the control values calculated by using the diseased plant rate (%) according to the following formula for other viruses (◎ : Excellent; ○: good; Δ; insufficient; and X: bad). The results are shown in Table 12.

Diseased plant rate (%) = (Number of diseased plants / Number of test plants) X 100

Control value (%) = [1-(Diseased plant rate of the treated plants / Diseased plant rate of the non-treated plants] X 100)

<Table 12>

| Virus | Host plant | Controlling activity | | |
|---|---|---|---|---|
| | | 250-fold dilution | 500-fold dilution | 1,000-fold dilution |
| Tobacco mosaic virus | Tobacco | ◎ | ◎ | ◎ |
| Cucumber green mottle mosaic virus | Watermelon | ○ | ○ | Δ |
| | Cucumber | ○ | ○ | Δ |
| Pepper mild mottle virus | Pepper | ◎ | ◎ | ◎ |
| Pepper mottle virus | Pepper | ◎ | ◎ | ◎ |
| Zucchini yellow mosaic virus | Zucchini | ◎ | ◎ | ◎ |
| Watermelon mosaic virus | Watermelon | ◎ | ○ | ○ |
| Potato virus Y | Cucumber | ◎ | ◎ | ◎ |
| | Tobacco | ◎ | ◎ | ◎ |
| Cucumber mosaic virus | Melon | ◎ | ○ | ○ |

<u><6-2> Evaluation of efficacy in the field on Tobacco mosaic virus</u>

In the test of controlling efficacy on the Pepper mottle virus in <6-1>, the control value of the KBPF-004 25% wettable powder after 4 weeks from the inoculation of the Tobacco mosaic virus was calculated, and the results are shown in Table 13. As a result, the control value of 500-fold and 1,000-fold dilutions was 90.8% and 76.8%, respectively, which are higher than that of powdered skim milk as a control. Therefore, the anti-viral active materials of the KBPF-004 strain show a controlling effect in a concentration dependent manner.

<Table 13>

| Group | Dilution ratio | Diseased plant rate (%) | | | | Significant difference (DMRT) | | Control value (%) |
|---|---|---|---|---|---|---|---|---|
| | | A | B | C | Average | | | |
| KBPF-004 25% WP | 500-fold | 3.8 | 1.9 | 1.9 | 2.5 | a | | 90.8 |
| | 1,000-fold | 15.4 | 0 | 3.8 | 6.4 | a | | 76.8 |
| Powdered skim milk | 10-fold | 23.5 | 29.3 | 15.4 | 22.7 | | b | 17.5 |
| Non-treated group | - | 32.7 | 25.0 | 25.0 | 27.6 | | b | |
| * Coefficient of variation (C.V.): 34.6 % | | | | | | | | |

<u><6-3> Evaluation of efficacy in the field on Pepper mottle virus</u>

In the test of controlling efficacy on the Pepper mottle virus in <6-1>, the control value of the KBPF-004 25% wettable powder was calculated and the results are shown in Table 14 and Fig. 7. The infection-inhibiting rates of 500-fold and 1,000-fold dilutions against pepper mottle virus were both 100%. In addition, in the non-treated group, the infection rate by the pepper mottle virus was 5.4% before treatment and increased to 11.8% after treatment. However, in the KBPF-004 25% wettable powder-treated group, the infection rate by the pepper mottle virus was decreased after treatment and the virus was not transmitted any more.

<Table 14>

| Group | Dilution ratio | Virus infection rate (%) | | Infection progress (%) | Infection inhibiting rate (%) |
|---|---|---|---|---|---|
| | | Before treatment | After treatment | | |
| KBPF-004 25% WP | 1: 500 | 13.3 | 9.5 | 0 | 100 |
| | 1: 1,000 | 11.8 | 11.8 | 0 | 100 |
| Non-treated group | - | 5.4 | 11.8 | 203.4 | 0 |

<6-4> Evaluation of efficacy of KBPF-004 strain on rice stripe virus in a greenhouse

In the test of controlling efficacy on the rice stripe virus in <6-1>, 500-fold and 1,000-fold dilution of the KBPF-004 25% wettable powder was respectively sprayed on the rice seedlings 1 day before the inoculation of viruliferous insects (*Laodelphax striatllus F*). After 14 days from the inoculation of the viruliferous insects, the control value was calculated. As can be seen from the results shown in Table 15 and Fig. 8, 500-fold dilution exhibited excellent controlling efficacy compared with the non-treated group.

<Table 15>

| Group | Dilution ratio | Disease severity (%) | | | | Significant difference (DMRT) | | Control value (%) |
|---|---|---|---|---|---|---|---|---|
| | | A | B | C | Average | | | |
| KBPF-004 25% WP | 500-fold | 4 | 4 | 2 | 3.3 | a | | 89.6 |
| | 1,000-fold | 16 | 28 | 10 | 18.0 | a | b | 43.8 |
| Non-treated group | - | 44 | 28 | 24 | 32.0 | | b | |
| * Coefficient of variation (C.V.): 51.3 % | | | | | | | | |

In addition, 500-fold dilution of the KBPF-004 25% wettable powder was directly sprayed on the viruliferous insects (*Laodelphax striatllus F*) and after 1 day therefrom, the insects were inoculated to the rice seedlings. The control value was determined as above. As shown in Table 16, the transmission rate of the rice stripe virus was significantly decreased.

<Table 16>

| Group | Dilution ratio | Disease severity (%) | | | | Significant difference (DMRT) | | Control value (%) |
|---|---|---|---|---|---|---|---|---|
| | | A | B | C | Average | | | |
| KBPF-004 25% WP | 500-fold | 0.5 | 0.3 | 0.1 | 0.3 | a | | 99.4 |
| Non-treated | - | 54 | 43 | 65 | 54.0 | | b | |
| * Coefficient of variation (C.V.): 28.9 % | | | | | | | | |

<6-5> Evaluation of efficacy of the KBPF-004 granules on Tobacco mosaic virus

In order to examine the controlling efficacy of the KBPF-004 2.5% granules obtained in Example <4-1> when treated to the soils, a tobacco plant infected by Tobacco mosaic virus was freeze-dried and ground to prepare an infected source. 1 g of the infected source thus obtained was mixed with 100 g of bed soils to prepare diseased soils. Then, 1 g of the KBPF-004 2.5% granules was well mixed with 100 g of the diseased soils, and the local lesion host plant, tobacco (*Nicotiana tabacum* cv. Xanthi nc) of three foliage leaf stage was transplanted thereto and cultivated in a greenhouse for 3 weeks.

As shown in Table 17 and Fig. 9, the tobacco seedlings withered in the non-treated group, but well grew like the healthy plants in the KBPF-004 2.5% granule-treated group.

<Table 17>

| Group | Treated amounts/ soil | Diseased plant rate (%) | | | | Significant difference (DMRT) | | Control value (%) |
|---|---|---|---|---|---|---|---|---|
| | | A | B | C | average | | | |
| KBPF-004 25% granules | 1g/100g | 2.3 | 3.2 | 3.4 | 3.0 | a | | 93.3 |
| Non-treated | - | 43.2 | 39.3 | 51.8 | 44.8 | | b | |
| * Coefficient of variation (C.V.): 18.4 % | | | | | | | | |

<u><6-6> Evaluation of efficacy in the field on Tomato yellow leaf curl virus</u>

500-fold dilution of the KBPF-004 70% AS obtained in Example <4-5> was mixed with the conventional insecticides, such as Dinotefuran® WP, Dinotefuran® WG, Spiromesifen® SC, Dichlorvos® EC and Amitraz+buprofezin EC, and the mixture was sprayed to the tomato plants. The resulting control value was 99.9%. In contrast, when Spinosad WG, Acetamiprid WP, Dinotefuran WG, Spiromesifen SC, and Emamectin benzoate EC was alternatively sprayed to the plants at intervals of 7 days, the calculated diseased plant rate was 80.2%.

<Table 18>

| Group | Dilution ratio | Diseased plant rate (%) | | | | Significant difference (DMRT) | | Control value (%) |
|---|---|---|---|---|---|---|---|---|
| | | A | B | C | Average | | | |
| KBPF-004 70% AS | 500-fold | 0.2 | 0.1 | 0.1 | 0.1 | a | | 99.9 |
| The conventional insecticides | - | 77.6 | 74.5 | 88.4 | 80.2 | | b | |
| * Coefficient of variation (C.V.): 18.4 % | | | | | | | | |

## Claims

1. A *Pseudomonas oleovorans* strain KBPF-004 (KCTC 10159BP) having a controlling activity against plant viral diseases.

2. A culture of the *Pseudomonas oleovorans* strain of claim 1.

3. A dried powder of the culture of claim 2.

4. The dried powder of claim 3, wherein the dried powder is prepared by freeze drying or spray drying.

5. A microbial agent for controlling plant viral diseases comprising *Pseudomonas oleovorans* strain KBPF-004 (KCTC 10159BP), a culture thereof, or a dried powder of the culture as an active ingredient.

6. The microbial agent of claim 5, which comprises 2.5 to 70% by weight of the *Pseudomonas oleovorans* strain, a culture thereof, or a dried powder of the culture; 2 to 30% by weight of a surfactant; and a residual amount of a carrier.

7. The microbial agent of claim 6, wherein the surfactant is an anionic surfactant, nonionic surfactant, or a mixture thereof.

8. The microbial agent of claim 6, wherein the carrier is selected from the group consisting of bentonite, talc, clay, kaolin, calcium carbonate, silica, pumice stone, diatomaceous earth, acidic white bole, zeolite, perlite, white carbon, ammonium sulfate, urea, glucose, dextrin, water, and a mixture thereof.

9. The microbial agent of claim 5, which is in a form selected from the group consisting of wettable powders (WP), water dispersible granules (WG), suspension concentrates (SC), granules, aqueous suspensions (AS), soluble powders (SP), water soluble granules (SG) and capsules.

10. The microbial agent of claim 5, wherein the plant viral disease is caused by a plant virus group selected from the group consisting of Allexivirus, Alfamovirus, Ampelovirus, Bymovirus, Begomovirus, Capillovirus, Carlavirus, Carmovirus, Caulimovirus, Closterovirus, Comovirus, Cucumovirus, Crinivirus, Cytorhabdovirus, Fabavirus, Flexiviridae, Foveavirus, Furovirus, Geminivirus, Hordeivirus, Ilarvirus, Luteovirus, Maculavirus, Nepovirus, Potexvirus, Potyvirus, Phytoreovirus, Polerovirus, Pomovirus, Sadwavirus, Taastrupvirus, Tenuivirus, Tobamovirus, Tobravirus, Tombusvirus, Tospovirus, Trichovirus, and a combination thereof.

## Patentansprüche

1. *Pseudomonas oleovorans* Stamm KBPF-004 (KCTC 10159BP) mit einer Aktivität zur Kontrolle virusbedingter Erkrankungen bei Pflanzen.

2. Kultur des *Pseudomonas oleovorans* Stamms nach Anspruch 1.

3. Getrocknetes Pulver der Kultur nach Anspruch 2.

4. Getrocknetes Pulver nach Anspruch 3, wobei das getrocknete Pulver durch Gefriertrocknen oder Sprühtrocknen hergestellt wird.

5. Mikrobielles Agens zur Kontrolle von virusbedingten Erkrankungen bei Pflanzen, umfassend *Pseudomonas oleovorans* Stamm KBPF-004 (KCTC 10159BP), eine Kultur davon oder ein getrocknetes Pulver dieser Kultur als Wirkstoff.

6. Mikrobielles Agens nach Anspruch 5, welches 2,5 bis 70 Gew.-% des *Pseudomonas oleovorans* Stamms, einer Kultur davon oder eines getrockneten Pulvers der Kultur, 2 bis 30 Gew.-% eines Tensids und eine Restmenge eines Trägerstoffes umfasst.

7. Mikrobielles Agens nach Anspruch 6, wobei das Tensid ein anionisches Tensid, nichtionisches Tensid oder eine Mischung davon ist.

8. Mikrobielles Agens nach Anspruch 6, wobei der Trägerstoff aus der Gruppe ausgewählt ist, bestehend aus Bentonit, Talkum, Ton, Kaolin, Kalziumcarbonat, Siliziumdioxid, Bimsstein, Kieselgur, saurer Porzellanerde, Zeolith, Perlit, weißem Karbon, Ammoniumsulfat, Harnstoff, Glucose, Dextrin, Wasser und einer Mischung davon.

9. Mikrobielles Agens nach Anspruch 5, welches sich in einer Form befindet, ausgewählt aus der Gruppe, bestehend aus benetzbaren Pulvern (WP, wettable powders), wasserdispergierbaren Granulaten (WG, water dispersible granules), Suspensionskonzentraten (SC, suspension concentrates), Granulaten, wässrigen Suspensionen (AS, aqueous suspensions), löslichen Pulvern (SP, soluble powders), wasserlöslichen Granulaten (SG, water soluble granules) und Kapseln.

10. Mikrobielles Agens nach Anspruch 5, wobei die virusbedingte Erkrankung bei Pflanzen durch eine Gruppe von Pflanzenviren verursacht wird, die aus der Gruppe ausgewählt sind, bestehend aus Allexivirus, Alfamovirus, Ampelovirus, Bymovirus, Begomovirus, Capillovirus, Carlavirus, Carmovirus, Caulimovirus, Closterovirus, Comovirus, Cucumovirus, Crinivirus, Cytorhabdovirus, Fabavirus, Flexiviridae, Foveavirus, Furovirus, Geminivirus, Hordeivirus, Ilarvirus, Luteovirus, Maculavirus, Nepovirus, Potexvirus, Potyvirus, Phytoreovirus, Polerovirus, Pomovirus, Sadwavirus, Taastrupvirus, Tenuivirus, Tobamovirus, Tobravirus, Tombusvirus, Tospovirus, Trichovirus und einer Kombination davon.

**Revendications**

1. Souche de *Pseudomonas oleovorans* KBPF-004 (KCTC 10159BP) ayant une activité de lutte contre des maladies virales végétales.

2. Culture de la souche de *Pseudomonas oleovorans* selon la revendication 1.

3. Poudre séchée de la culture selon la revendication 2.

4. Poudre séchée selon la revendication 3, dans laquelle la poudre séchée est préparée par lyophilisation ou par séchage par atomisation.

5. Agent microbien pour lutter contre des maladies virales végétales comprenant la souche *Pseudomonas oleovorans* KBPF-004 (KCTC 10159BP), une culture de celle-ci, ou une poudre séchée de la culture en tant qu'ingrédient actif.

6. Agent microbien selon la revendication 5, qui comprend 2,5 à 70 % en poids de la souche de *Pseudomonas oleovorans,* une culture de celle-ci, ou une poudre séchée de la culture ; 2 à 30 % en poids d'un agent de surface ; et une quantité résiduelle d'un vecteur.

7. Agent microbien selon la revendication 6, dans lequel l'agent de surface est un agent de surface anionique, un agent de surface non ionique, ou un mélange de ceux-ci.

8. Agent microbien selon la revendication 6, dans lequel le vecteur est sélectionné à partir du groupe constitué par la bentonite, le talc, l'argile, le kaolin, le carbonate de calcium, la silice, la pierre ponce, la terre d'infusoires, le bol blanc acide, la zéolite, la perlite, le carbone blanc, le sulfate d'ammonium, l'urée, le glucose, la dextrine, l'eau et un mélange de ceux-ci.

**9.** Agent microbien selon la revendication 5, qui est dans une forme sélectionnée à partir du groupe constitué par des poudres mouillables (WP), des granules pouvant être dispersés dans l'eau (WG), des concentrés en suspension (SC), des granules, des suspensions aqueuses (AS), des poudres solubles (SP), des granules solubles dans l'eau (SG) et des capsules.

**10.** Agent microbien selon la revendication 5, dans lequel la maladie virale végétale est provoquée par un groupe de virus végétaux sélectionnés à partir du groupe constitué par Allexivirus, Alfamovirus, Ampélovirus, Bymovirus, Begomovirus, Capillovirus, Carlavirus, Carmovirus, Caulimovirus, Closterovirus, Comovirus, Cucumovirus, Crinivirus, Cytorhabdovirus, Fabavirus, Flexiviridae, Foveavirus, Furovirus, Geminivirus, Hordeivirus, Ilarvirus, Lutéovirus, Maculavirus, Nepovirus, Potexvirus, Potyvirus, Phytoréovirus, Polérovirus, Pomovirus, Sadwavirus, Taastrupvirus, Tenuivirus, Tobamovirus, Tobravirus, Tombusvirus, Tospovirus, Trichovirus et une combinaison de ceux-ci.

FIG. 1

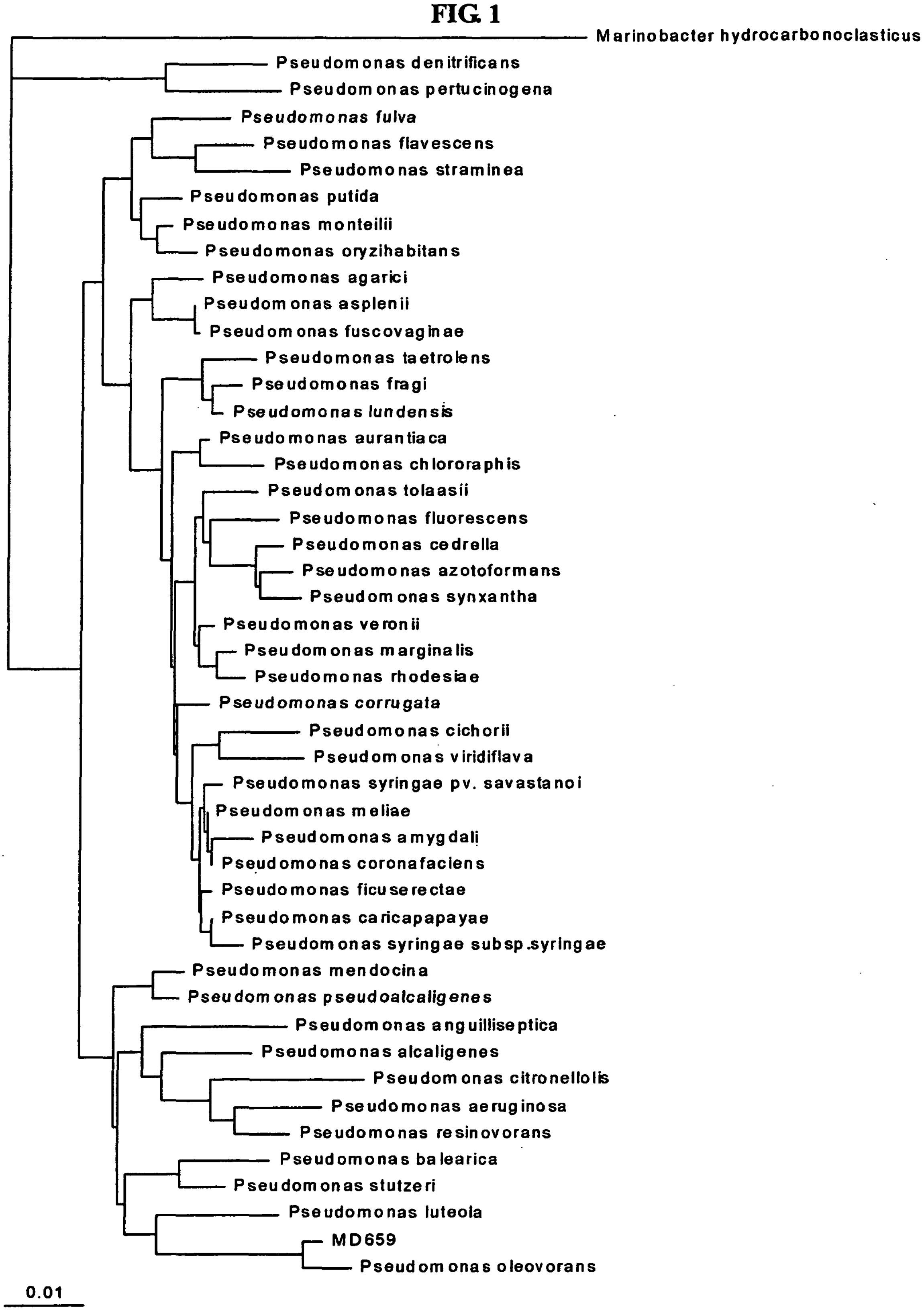
Marinobacter hydrocarbonoclasticus
Pseudomonas denitrificans
Pseudomonas pertucinogena
Pseudomonas fulva
Pseudomonas flavescens
Pseudomonas straminea
Pseudomonas putida
Pseudomonas monteilii
Pseudomonas oryzihabitans
Pseudomonas agarici
Pseudomonas asplenii
Pseudomonas fuscovaginae
Pseudomonas taetrolens
Pseudomonas fragi
Pseudomonas lundensis
Pseudomonas aurantiaca
Pseudomonas chlororaphis
Pseudomonas tolaasii
Pseudomonas fluorescens
Pseudomonas cedrella
Pseudomonas azotoformans
Pseudomonas synxantha
Pseudomonas veronii
Pseudomonas marginalis
Pseudomonas rhodesiae
Pseudomonas corrugata
Pseudomonas cichorii
Pseudomonas viridiflava
Pseudomonas syringae pv. savastanoi
Pseudomonas meliae
Pseudomonas amygdali
Pseudomonas coronafaciens
Pseudomonas ficuserectae
Pseudomonas caricapapayae
Pseudomonas syringae subsp.syringae
Pseudomonas mendocina
Pseudomonas pseudoalcaligenes
Pseudomonas anguilliseptica
Pseudomonas alcaligenes
Pseudomonas citronellolis
Pseudomonas aeruginosa
Pseudomonas resinovorans
Pseudomonas balearica
Pseudomonas stutzeri
Pseudomonas luteola
MD659
Pseudomonas oleovorans
0.01

FIG. 2

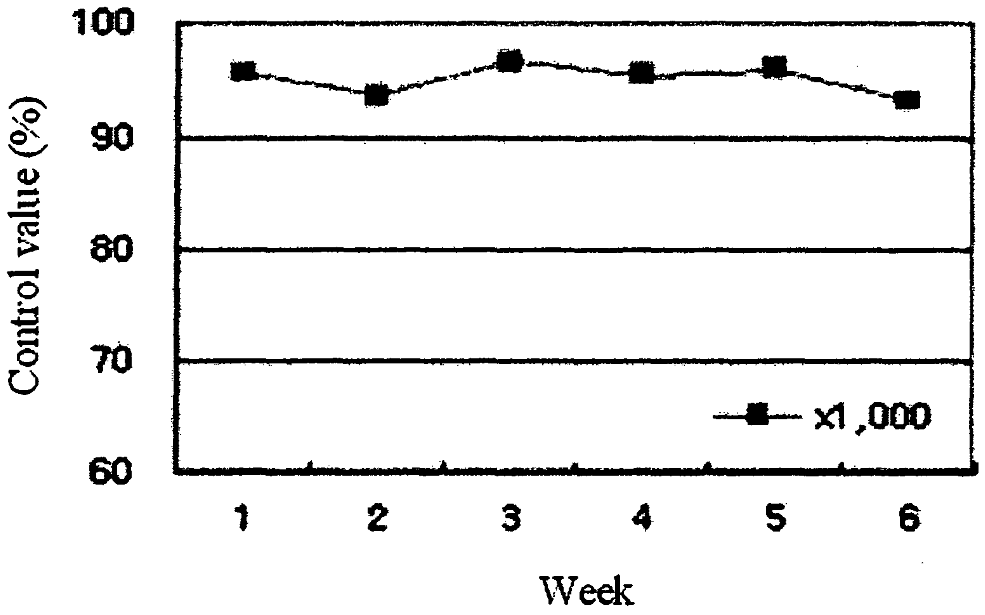


FIG. 3

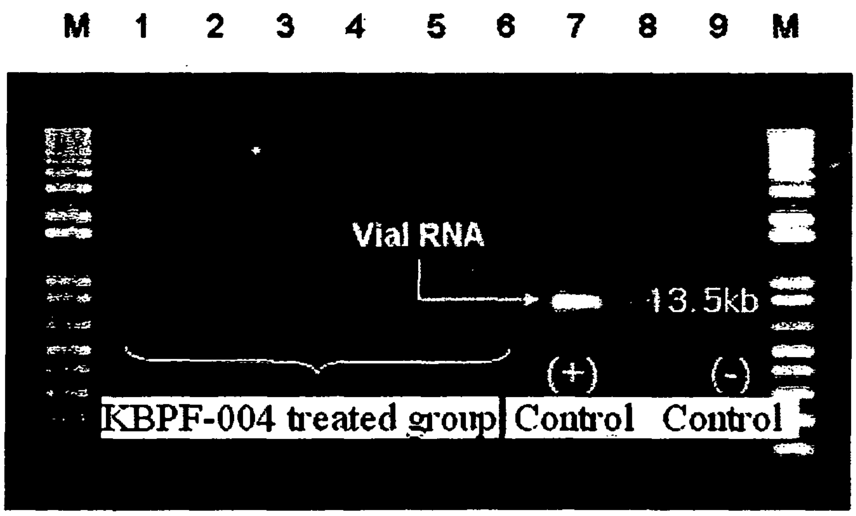


M: 1kb plus ladder
1: KBPF-004A 0.02g/ml
2: KBPF-004A 0.005g/ml
3: KBPF-004B 0.01g/ml
7: Mock
9: Healthy plant (-)
2: KBPF-004A 0.01g/ml
4: KBPF-004B 0.02g/ml
6: KBPF-004B 0.005g/ml
8: TMV-ul(+)

FIG. 4

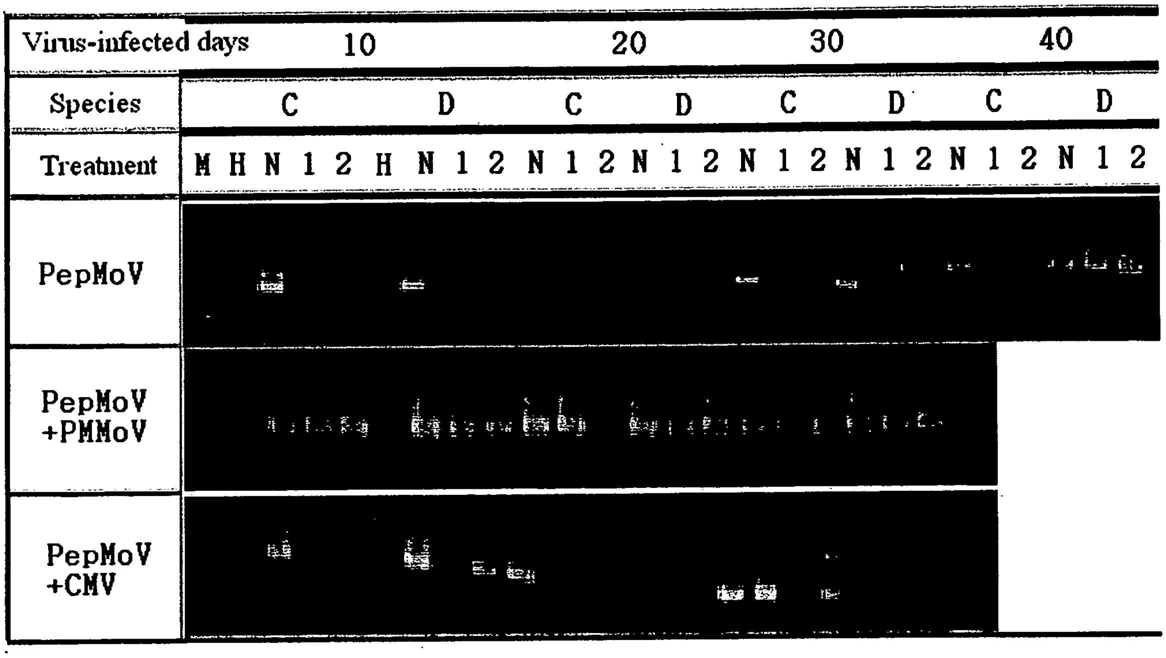
Virus-infected days
10
20
30
40
Species
C D C D C D C D
Treatment
M H N 1 2 H N 1 2 N 1 2 N 1 2 N 1 2 N 1 2 N 1 2 N 1 2
PepMoV
PepMoV +PMMoV
PepMoV +CMV

FIG. 5

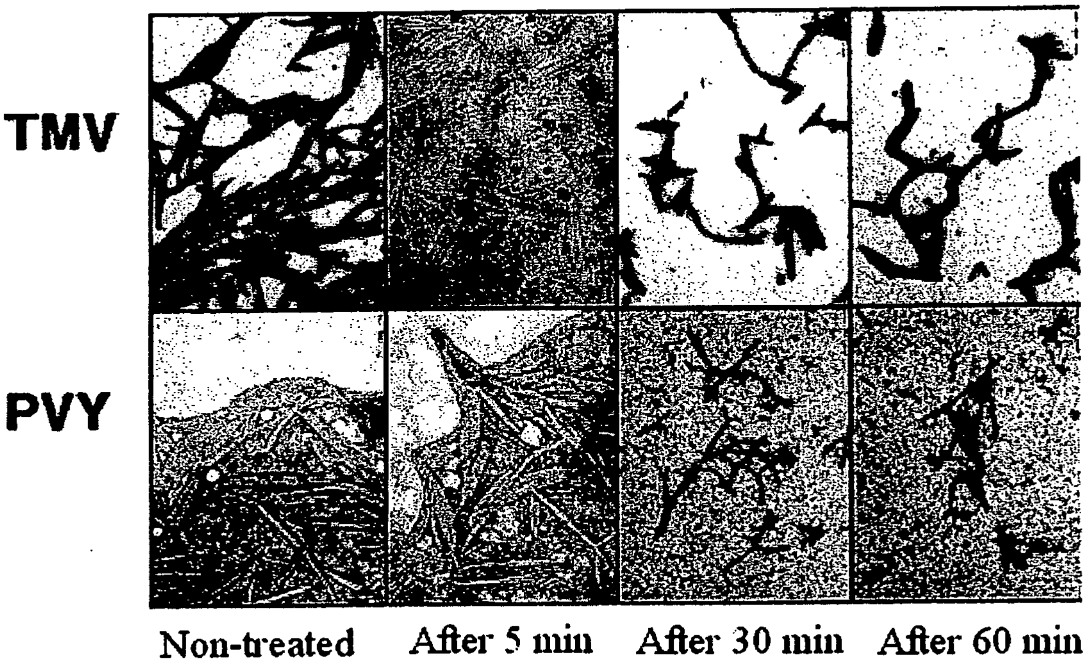
TMV
PVY
Non-treated
After 5 min
After 30 min
After 60 min

FIG. 6

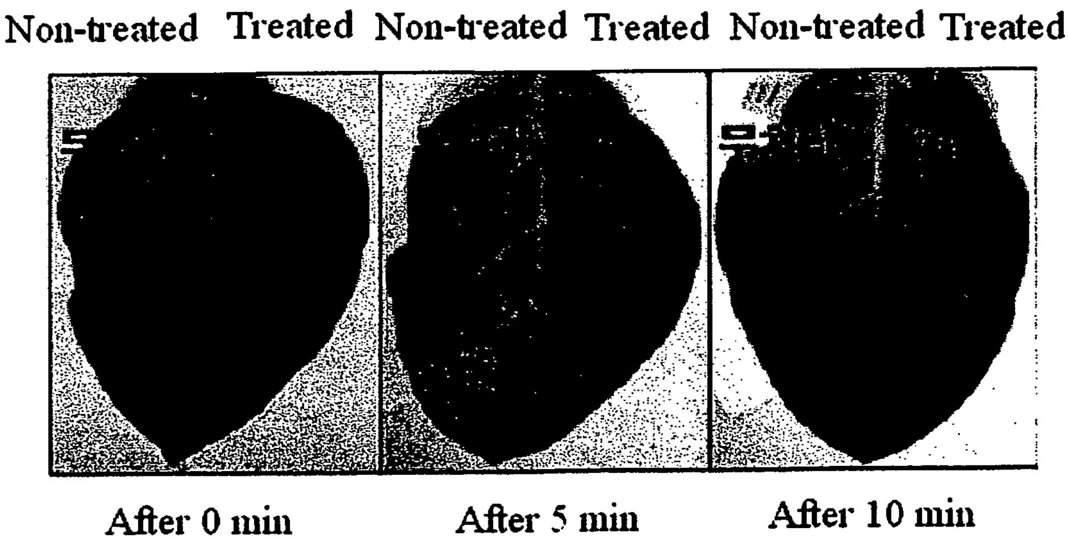
Non-treated
Treated
Non-treated
Treated
Non-treated
Treated
After 0 min
After 5 min
After 10 min

FIG. 7

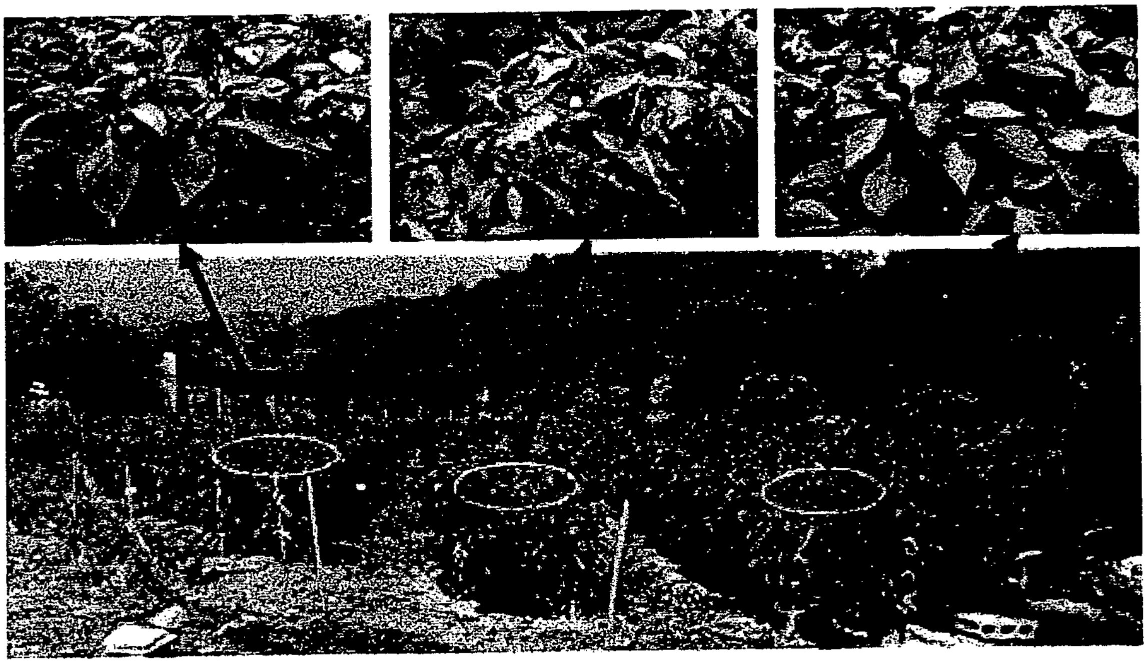

KBPF-004
500-fold dilution

Non-treated group

KBPF-004
1,000-fold dilution

FIG. 8

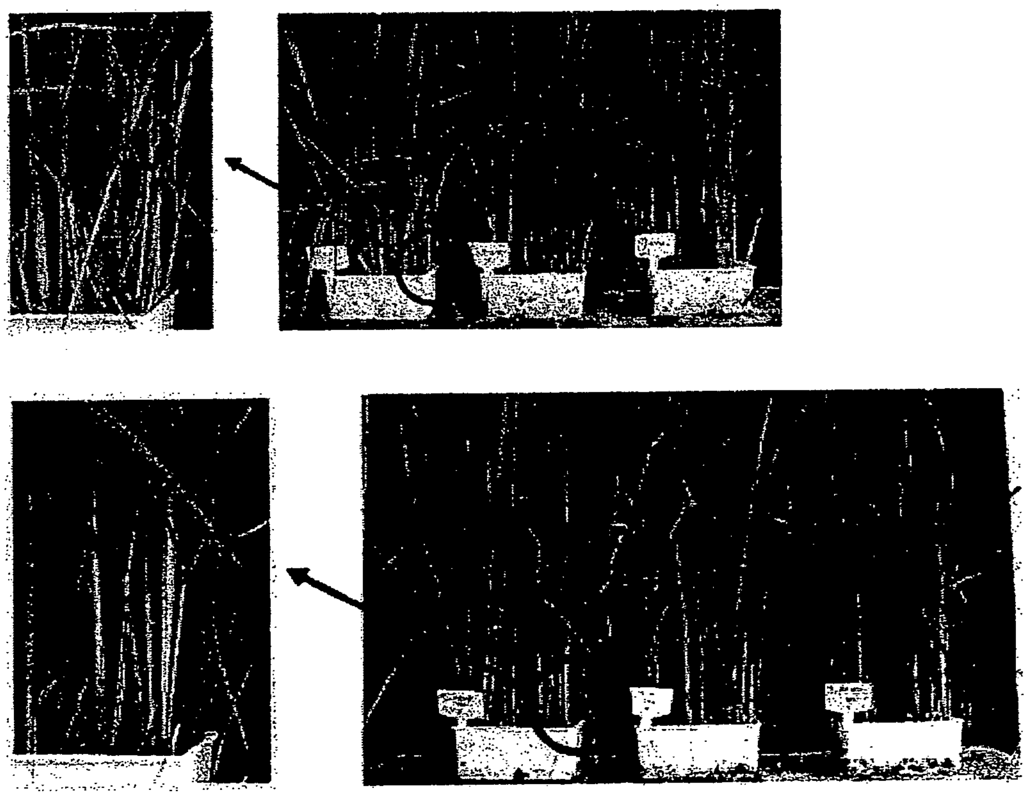

A

B

FIG. 9

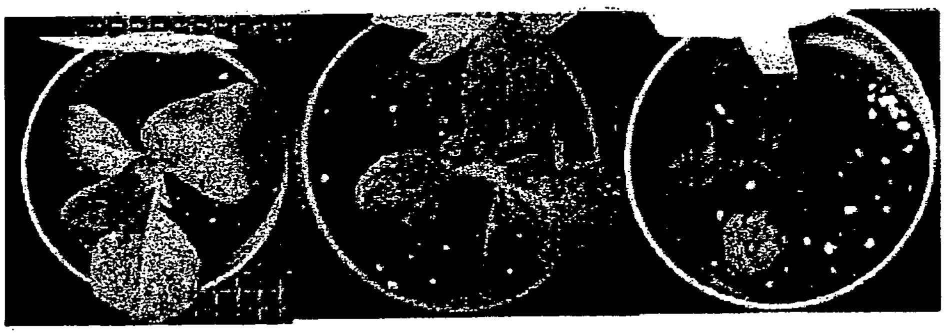

Healthy plant | KBPF-004 2.5% granule-treated group | Non-treated group

FIG. 10A

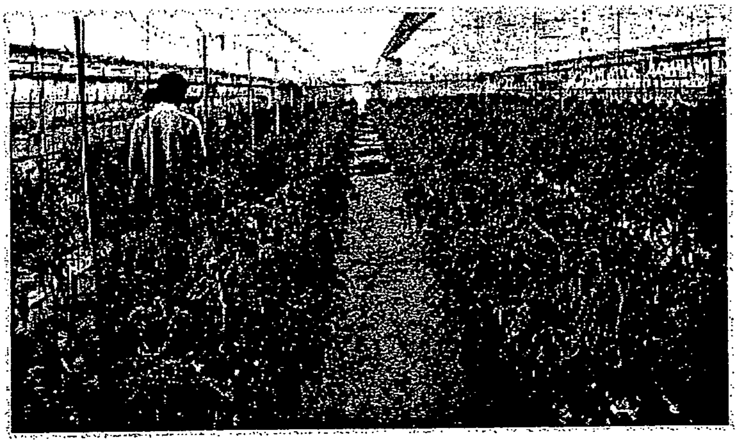

FIG. 10B

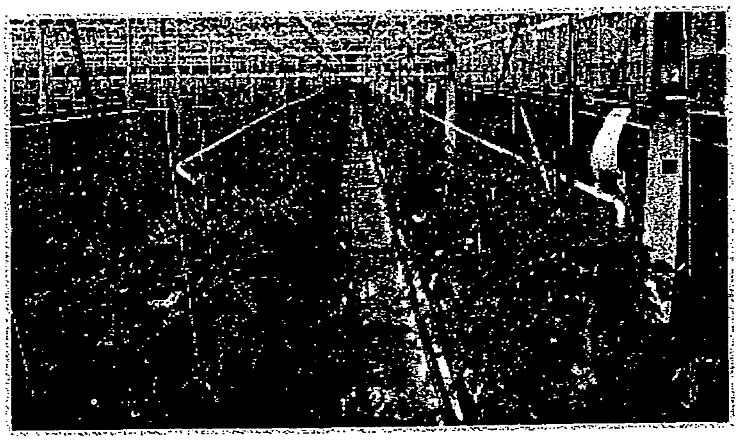

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 09830560 A
- KR 2008120899
- KR 2009007098 W


### Non-patent literature cited in the description


- **FITCHEN, J. H. ; BEACHY, R. N.** *Annu. Rev. Microbiol.,* 1993, vol. 47, 739-763
- **THOMPSON JD et al.** *Nuc. Acid. Res.,* 1997, vol. 25, 4876-82
- **KIM et al.** *Plant Pathol. J.,* 2004, vol. 20 (4), 293-296
- **CHOI et al.** *Plant Pathol. J.,* 1998, vol. 14, 7-12
- **YOON.** *Doctoral Thesis, Seoul Women's Univ., Seoul,* 2003, 166
- **CLARK et al.** *J. Gen. Virol.,* 1977, vol. 34, 475-483

<110> LEE, YONG JIN

<120> PSEUDOMONAS OLEOVORANS STRAIN CONTROLLING PLANT VIRUS DISEASES

<130> 463.109537

<140> EP09830560.0
<141> 2009-12-01

<150> KR2008-120899
<151> 2008-12-02

<150> PCT/KR2009/007098
<151> 2009-12-01

<160> 1

<170> KopatentIn 1.71

<210> 1
<211> 1479
<212> DNA
<213> Pseudomonas oleovorans

<400> 1

```
tggcggcagg cctaacacat gcaagtcgag cggatgagag gagcttgctc ctcgattcag        60
cggcggacgg gtgagtaatg cctaggaatc tgcctagtag tgggggacaa cgtttcgaaa       120
ggaacgctaa taccgcatac gtcctacggg agaaagtggg ggatcttcgg acctcacgct       180
attagatgag cctaggtcgg attagctagt tggtagggta aaggcctacc aaggcgacga       240
tccgtaactg gtctgagagg atgatcagtc acactggaac tgagacacgg tccagactcc       300
tacgggaggc agcagtgggg aatattggac aatgggcgaa agcctgatcc agccatgccg       360
cgtgtgtgaa gaaggccttc gggttgtaaa gcactttaag ttgggaggaa gggcttacag       420
cgaatacctg tgagttttga cgttaccgac agaataagca ccggctaact tcgtgccagc       480
agccgcggta atacgaaggg tgcaagcgtt aatcggaatt actgggcgta aagcgcgcgt       540
aggtggcttg ataagttgga tgtgaaatcc ccgggctcaa cctgggaact gcatccaaaa       600
ctgtctggct agagtgtggt agagggtagt ggaatttcca gtgtagcggt gaaatgcgta       660
gatattggaa ggaacaccag tggcgaaggc gactacctgg actaacactg acactgaggt       720
gcgaaagcgt ggggagcaaa caggattaga taccctggta gtccacgccg taaacgatgt       780
caactagccg ttgggatcct tgagatctta gtggcgcagc taacgcatta agttgaccgc       840
ctggggagta cggccgcaag gttaaaactc aaatgaattg acgggggccc gcacaagcgg       900
tggagcatgt ggtttaattc gaagcaacgc gaagaacctt acctggcctt gacatgctga       960
gaactttcca gagatggatt ggtgccttcg ggaactcaga cacaggtgct gcatggctgt      1020
cgtcagctcg tgtcgtgaga tgttgggtta agtcccgtaa cgagcgcaac ccttgtcctt      1080
```

```
agttaccagc acgttatggt gggcactcta aggagactgc cggtgacaaa ccggaggaag    1140
gtggggatga cgtcaagtca tcatggccct tacggccagg gctacacacg tgctacaatg    1200
gtcggtacaa agggttgcca agccgcgagg tggagctaat cccataaaac cgatcgtagt    1260
ccggatcgca gtctgcaact cgactgcgtg aagtcggaat cgctagtaat cgtgaatcag    1320
aacgtcacgg tgaatacgtt cccgggcctt gtacacaccg cccgtcacac catgggagtg    1380
ggttgctcca gaagtagcta gtctaacctt cgggaggacg gttaccacgg agtgattcat    1440
gactgggtga agtcgtaaca aggtagccgt aggggaacc                           1479
```